# EUROPEAN PATENT APPLICATION

(11) **EP 2 087 905 A2**
(43) Date of publication of application: **12.08.2009**
(21) Application number: 09158227.0
(22) Date of filing: 09.08.2004
(51) Int. Cl.: A61K 38/20, G01N 33/53

(54) **Interleukin-20 for treating and diagnosing conditions associated with neovascularisation**

(30) Priority: 08.08.2003 DK 200301151; 14.08.2003 US 495300 P
(62) Divisional of application: 04739029.9
(71) Applicant: NOVO NORDISK A/S, 2880 Bagsvaerd (DK)
(72) Inventor: Olsen, Uffe Bang, 2625 Vallensbæk (DK); Hansen, Anker Jon, 2920 Charlottenlund (DK); Rømer, John, 2100 København (DK); Hasselager, Erik, 3250 Gilleleje (DK); Clausen, Jes Thorn, 4270 Høng (DK); Iversen, Lars Fogh, 2840 Holte (DK)

(57) **Abstract**

The invention provides a novel method of regulating neovascularisation.

## Description

### FIELD OF THE INVENTION

IL-20 acts as an angiogenic factor in the body. Thus, the present invention relates to the IL-20, analogues and variants and derivatives thereof for facilitating neovascularisation in tissue.

Inhibition/neutralization of IL-20 by therapeutic antibodies or soluble receptors or by blocking related receptors by small molecules or antisense or sRNAi can prevent growth of tumours and treatment of inflammatory diseases. Also, IL-20 or analogues of IL-20 can be used for treatment in wound healing, skin ulceration or organ regeneration caused by trauma and ischemia.

### BACKGROUND OF THE INVENTION

The present invention relates to the finding that IL-20 has an effect in neovascularisation. US6610286 indicates that IL-20 has an effect on IL-8, which acts angiogenic. However, results indicate that IL-20 itself has an effect on angiogenesis independently of IL-8. A direct control of the angiogenetic effect is thus possible.

Abnormal blood vessel growth affects many diseases, including chronic inflammation and cancer. For example, neovascularisation associated with psoriasis contributes to the increasing turnover of epithelial cells and puritic plaques; it contributes to Crohn's disease by providing a way for inflammatory cells to enter sites of injury; it is part of the pannus, the excessive folds of inflamed tissue, in rheumatoid arthritis; and it is the basis for intraperitoneal bleeding in endometriosis and for macular degeneration in diabetic retinopathy. Obviously remedies or treatments that counteract neovascularisation should be of therapeutic value in this kind of diseases.

On the other hand the remedies that promote neovascularisation are of therapeutic value in a number of diseases. These factors may be used to revive damaged tissue following ischemic or traumatic insults in CNS or organs, e.g. in the myocardium; or to accelerate epithelial repair after lesions in surface membranes to mention a few. (Neovascularisation and some potential implications of angiogenesis are discussed in WO99/55869).

In summary neovascularisation is normally observed in embryonal and fetal development, in formation of the corpus luteum, endometrium and in wound healing. It is involved in disorders like diabetic retinopathy, macular degeneration, atherosclerosis, psoriasis, rheumatoid arthritis and tumor growth. Neovascularisation is also suggested as an early indicator of cervical cancer. Obesity, diabetes and perhaps even Alzheimer also depend on neovascularisation. Hence anti-neovascularisation may be effective for several of the diseases mentioned.

Because neovascularisation in an adult healthy animal is limited to wound healing and the female reproductive cycle, it is a specific indicator of a pathological process such as development of solid tumors and inflammatory conditions e.g. retinopathies and arthritis. The invention thus also provides methods useful in diagnosing diseases and disorders which is related to neovascularisation.

### SUMMARY OF THE INVENTION

The invention provides in a first embodiment the use of IL-20 for the manufacture of a medicament for the treatment of conditions which would benefit from improved neovascularisation.

The invention provides the use of an antagonist of IL-20 for the manufacture of a medicament for the treatment of conditions which would benefit from reduced neovascularisation.

The invention also provides a pharmaceutical composition comprising IL-20 together with pharmaceutically acceptable carriers and diluents.

The invention also provides a pharmaceutical composition comprising an antagonist of IL-20 together with pharmaceutically acceptable carriers and diluents.

The invention also provides a method of treating a mammal in need of neovascularisation with an effective amount of IL-20.

The invention also provides a method of treating a mammal in need of reduced neovascularisation with an effective amount of an antagonist of IL-20.

The invention also provides a method for diagnosing neovascularisation activity.

### DESCRIPTION OF THE DRAWINGS

Fig. 1 shows a graphic representation of the amount of hemoglobin in matrigel.
Fig. 2 shows histological examinations showing vessel formations in the matrigel
Fig. 3 shows the induction of neovascularisation in mouse cornea by IL-20.
Fig. 4 shows the quantification of the above experiments.
Fig. 5 shows dose-dependent chemotactic effect on HUVEC in a modified Boyden chamber assay.

### DEFINITIONS

Prior to a discussion of the detailed embodiments of the invention, a definition of specific terms related to the main aspects of the invention is provided.

"Neovascularisation" refers to formation of new blood vessels in tissues/organs. It includes several distinct processes including "angiogenesis", "vasculogenesis", and "intussuception". "Angiogenesis" is the process of sprouting of new blood vessels from preexisting blood vessels. "Vasculogenesis" is the process of de novo formation of blood vessels due to differentiation of stem cells into vascular endothelial cells.

"Intussuception" is the process of division of a large "mother" vessel into smaller "daughter" vessels by growing interstitial tissues into the lumen of the large vessel.

In accordance with the present invention there may be employed conventional molecular biology, microbiology, and recombinant DNA techniques within the skill of the art. Such techniques are explained fully in the literature. See, e.g., Sambrook, Fritsch & Maniatis, Molecular Cloning: A Laboratory Manual, Second Edition (1989) Cold Spring Harbor Laboratory Press, Cold Spring Harbor, New York (herein "Sambrook et al., 1989") DNA Cloning: A Practical Approach, Volumes I and II /D.N. Glover ed. 1985); Oligonucleotide Synthesis (M.J. Gait ed. 1984); Nucleic Acid Hybridization (B.D. Hames & S.J. Higgins eds (1985)); Transcription And Translation (B.D. Hames & S.J. Higgins, eds. (1984)); Animal Cell Culture (R.I. Freshney, ed. (1986)); Immobilized Cells And Enzymes (IRL Press, (1986)); B. Perbal, A Practical Guide To Molecular Cloning (1984).

An "effective amount" means an amount that is sufficient for inducing neovascularisation. It will depend on the means of administration, target site, state of the patient, whether the treatment takes place in the subject or on isolated cells, the frequency of treatment etc. Dosage ranges would ordinarily be expected from 1 microgram to 1000 microgram per kilogram of body weight per day. For a complete discussion of drug formulations and dosage ranges see Remington's Pharmaceutical Sciences, 18th Ed., (Mack Publishing Co., Easton, Penn., 1996).

In the context of the present invention "treatment" or "treating" refers to preventing, alleviating, curing or reducing the disease. As the present invention relates to neovascularisation, which may be a beneficial effect in some instances and not desirable in other disorders, the term "treatment" or "treating" may in the context of this invention refer to as well a reduction of the observed effect as well as an induction of the effect.

In the context of the present invention "cancer" refers to any neoplastic disorder, including such cellular disorders as for example sarcoma, carcinoma, melanoma, leukemia, lymphoma, cancers in the breast, head and neck, ovaries, bladder, lung, pharynx, larynx, oesophagus, stomach, small intestines, liver, pancreas, colon, female reproductive tract, male reproductive tract, prostate, kidneys and central nervous system. In an aspect of the invention the cancers are in the skin, prostate, pancreas, lung, ovary or testis.

In the context of this invention "IL-20" is defined in WO9927103, which comprises the following:
IL-20 comprises 152 amino acids (SEQ ID No:1):
   LKTLNLGSCVIATNLQEIRNGFSDIRGSVQAKDGNIDIRILRRTESLQDTKPANRCCLLR
   HLLRLYLDRVFKNYQTPDHYTLRKISSLANSFLTIKKDLRLCHAHMTCHCGEEAMKKYSQ
   ILSHFEKLEPQAAVVKALGELDILLQWMEETE

IL-20 also comprises the following sequence (SEQ 2), which additionally comprises a signal sequence in the N-terminal:
MKASSLAFSLLSAAFYLLWTPSTGLKTLNLGSCVIATNLQEIRNGFSDIRGSVQAKDGNI
DIRILRRTESLQDTKPANRCCLLRHLLRLYLDRVFKNYQTPDHYTLRKISSLANSFLTIK
KDLRLCHAHMTCHCGEEAMKKYSQILSHFEKLEPQAAVVKALGELDILLQWMEETE

"IL-20" thus comprises the sequences above and includes the protein as well as analogues, active fragments and derivatives thereof, having an activity as described in the present invention.

### DESCRIPTION OF THE INVENTION

The present invention provides novel uses of IL-20 and antagonists of IL-20. IL-20 is involved directly in neovascularisation. As such IL-20 can be used in the treatment of diseases wherein neovascularisation is favorable. Antagonists of IL-20 is thus useful in conditions wherein prevention of neovascularisation is preferred.

Diseases wherein neovascularisation is involved are for example: conditions relating to ischemia, transplants of tissue, grafting of skin, tissue or organs, repair of epithelial tissue, endometriosis, various ocular diseases such as diabetic retinopathy, retinopathy of prematurity, corneal graft rejection, retrolental fibroplasia, neovascular glaucoma, rubeosis, macular degeneration, hypoxia, and other abnormal neovascularisations of the eye, obesity, treatment of primary tumors and prevention of metastasis or spread of cancer, in birth control by inhibiting ovulation and the development of a placenta, inflammatory diseases such as for example rheumatoid arthritis, blood vessel diseases such as hemagiomas and capillary proliferation within atherosclerotic plaques, mycardial angiogenesis, angiofibroma, neovascularisation of plaques, formation of hypertrophic scars for example keloids."IL-20" denotes a four helix bundle cytokine, IL-20, encoded by the IL-20 gene located on the human chromosome 1q32.2 (Acc No. Q9NYY1, EMBL AF224266/AF402002/ AAF36679.1/AAK84423.1) and is described in International Patent Application No. PCT/US98/25228, publication no. WO 99/27103, published June 3, 1999, which is hereby incorporated in this application in its entirety, discloses IL-20 (as "Zcyto 10") as SEQ ID No. 2, which is hereby incorporated in this application in its entirety, as well as methods for producing it and antibodies thereto and a polynucleotide sequence encoding IL-20 as SEQ ID No. 1 in the aforementioned application. The present invention also contemplates the use of IL-20 polypeptides which as used herein should be taken to mean polypeptides with a sequence identity to the polypeptide of SEQ ID No: 1 and SEQ ID No:2, as mentioned above in the present application, which denotes the peptide sequence without the signal sequence as SEQ ID No: 1 and including the signal sequence as SEQ ID No:2. The invention comprises their orthologs comprising at least 70%, at least 80%, at least 90%, at least 95%, or greater than 95%. The present invention also includes the use of polypeptides that comprise an amino acid sequence having at least 70%, at least 80%, at least 90%, at least 95% or greater than 95% sequence identity to the sequence of amino acid residues 1 to 176, residues 25 to 151, or residues 33 to 176 of SEQ ID No: 2 Methods for determining percent identity are described below. The IL-20 polypeptides of the present invention have retained all or some of the biological activity of IL-20 which makes IL-20 useful for treating cancer. Some of the polypeptides may also have a biological activity which is higher than the biological activity of IL-20.

The present invention embraces counterpart proteins and polynucleotides from other species ("species orthologs"). Of particular interest are IL-20 polypeptides from other mammalian species, including murine, porcine, ovine, bovine, canine, feline, equine, and other primates. Species orthologs of the human IL-20 protein can be cloned using information and compositions provided by the present invention in combination with conventional cloning techniques. As used and claimed, the language "an isolated polynucleotide which encodes a polypeptide, said polynucleotide being defined by SEQ ID NOs: 2" includes all allelic variants and species orthologs of this polypeptide.

The present invention also provides isolated protein polypeptides that are substantially identical to the protein polypeptide of SEQ ID NO: 2 of WO99/27103 and its species orthologs. By "isolated" is meant a protein or polypeptide that is found in a condition other than its native environment, such as apart from blood and animal tissue. In a preferred form, the isolated polypeptide is substantially free of other polypeptides, particularly other polypeptides of animal origin. It is preferred to provide the polypeptides in a highly purified form, i.e. greater than 95% pure, more preferably greater than 99% pure. The term "substantially identical" is used herein to denote polypeptides having 50%, preferably 60%, more preferably at least 80%, sequence identity to the sequence shown in SEQ ID NOs: 2 of WO99/27103 or species orthologs. Such polypeptides will more preferably be at least 90% identical, and most preferably 95% or more identical to SEQ ID N0:2 of WO99/27103 or its species orthologs. Percent sequence identity is determined by conventional methods. See, for example, Altschul et al., Bull. Math. Bio. 48: 603 616 (1986) and Henikoff and Henikoff, Proc. Natl. Acad. Sci. USA 89:10915 10919 (1992). Sequence identity of polynucleotide molecules is determined by similar methods using a ratio as disclosed above.

Antagonists or inhibitors of IL-20, analogues, active fragments or derivatives thereof, are useful in the treatment of diseases of which neovascularisation is not considered beneficial. Inhibitors of IL-20 activity (antagonist) include anti-IL-20 antibodies and soluble receptors, as well as other peptidic and non-peptidic agents.

Antibodies are produced by the methods described in US 6,486,301, column 69-70 (production of antibodies)

IL-20 excerts and signal its effect through the IL-20R1/IL-20R2 heterodimer and are also ligand for the receptor complex composed of IL-20R2 and IL-22R1 (Dumoutier et al, J. Immol 167:3545-3549, 2001; Fickenscher et al, Trends Immunol 23:89-96, 2002).

The invention includes administration of the soluble parts of each receptor or as a complex of receptors such as described in WO01/46232.

Antagonists may also be small-molecules or receptor binding fragments of IL-20, which are otherwise inactive.

Variant IL-20 polypeptides or substantially identical proteins and polypeptides are characterized as having one or more amino acid substitutions, deletions or additions. These changes are preferably of a minor nature, that is conservative amino acid substitutions (see Table 1) and other substitutions that do not significantly affect the folding or activity of the protein or polypeptide; small deletions, typically of one to about 30 amino acids; and small amino or carboxyl terminal extensions, such as an amino terminal methionine residue, a small linker peptide of up to about 20 25 residues, or a small extension that facilitates purification (an affinity tag), such as a polyhistidine tract, protein A, Nilsson et al., EMBO J. 4:1075 (1985); Nilsson et al., Methods Enzymol. 198:3 (1991), glutathione S transferase, Smith and Johnson, Gene 67:31 (1988), or other antigenic epitope or binding domain. See, in general Ford et al., Protein Expression and Purification 2: 95 107 (1991). DNAs encoding affinity tags are available from commercial suppliers (e.g., Pharmacia Biotech, Piscataway, NJ).

**Table 1**

| Conservative amino acid substitutions | |
|---|---|
| Basic: | arginine |
| | lysine |
| | histidine |
| Acidic: | glutamic acid |
| | aspartic acid |
| Polar: | glutamine |
| | asparagine |
| Hydrophobic: | leucine |
| | isoleucine |
| | valine |
| Aromatic: | phenylalanine |
| | tryptophan |
| | tyrosine |
| Small: | glycine |
| | alanine |
| | serine |
| | threonine |
| | methionine |

The proteins of the present invention can also comprise non naturally occurring amino acid residues. Non naturally occurring amino acids include, without limitation, trans 3 methylproline, 2,4 methanoproline, cis 4 hydroxyproline, trans 4 hydroxyproline, Nmethylglycine, addo threonine, methylthreonine, hydroxyethylcysteine, hydroxyethylhomo-cysteine, nitroglutamine, homoglutamine, pipecolic acid, thiazolidine carboxylic acid, dehy-droproline, 3 and 4-methylproline, 3,3 dimethylproline, tert leucine, norvaline, 2 azaphenylalanine, 3 azaphenylalanine, 4-azaphenylalanine, and 4 fluorophenylalanine. Several methods are known in the art for incorporating nonnaturally occurring amino acid residues into proteins. For example, an in vitro system can be employed wherein nonsense mutations are suppressed using chemically aminoacylated suppressor tRNAs. Methods for synthesizing amino acids and aminoacylating tRNA are known in the art. Essential amino acids in the polypeptides of the present invention can be identified according to procedures known in the art, such as site directed mutagenesis or alaninescanning mutagenesis [Cun-ningham and Wells, Science 244: 1081 1085 (1989)]; Bass et al., Proc. Natl. Acad. Sci. USA 88:4498 4502 (1991). In the latter technique, single alanine mutations are introduced at every residue in the molecule, and the resultant mutant molecules are tested for biological activity (e.g., ligand binding and signal transduction) to identify amino acid residues that are critical to the activity of the molecule. Sites of ligand protein interaction can also be determined by analysis of crystal structure as determined by such techniques as nuclear magnetic resonance, crystallography or photoaffinity labeling. See, for example, de Vos et ad., Science 255:306 312 (1992); Smith et al., J. Mod. Biod. 224:899 904 (1992); Wlodaver et ad., FEES Lett. 309:59 64 (1992). The identities of essential amino acids can also be inferred from analysis of homologies with related proteins.

Multiple amino acid substitutions can be made and tested using known methods of mutagenesis and screening, such as those disclosed by Reidhaar Olson and Sauer, Science 241:53 57 (1988) or Bowie and Sauer Proc. Nat1. Acad. Sci. USA 86:2152 2156 (1989). Briefly, these authors disclose methods for simultaneously randomizing two or more positions in a polypeptide, selecting for functional polypeptide, and then sequencing the mutagenized polypeptides to determine the spectrum of allowable substitutions at each position. Other methods that can be used include phage display (e.g., Lowman et al., Biochem.30:10832 10837 (1991); Ladner et al., U.S. Patent No. 5,223,409; Huse, WIPO Publication WO 92/06204) and region directed mutagenesis, Derbyshire et al., Gene 46:145 (1986); Ner et al., DNA 7:127 (1988).

Mutagenesis methods as disclosed above can be combined with high throughput screening methods to detect activity of cloned, mutagenized proteins in host cells. Preferred assays in this regard include cell proliferation assays and biosensor based ligand binding assays, which are described below. Mutagenized DNA molecules that encode active proteins or portions thereof (e.g., ligand binding fragments) can be recovered from the host cells and rapidly sequenced using modern equipment. These methods allow the rapid determination of the importance of individual amino acid residues in a polypeptide of interest, and can be applied to polypeptides of unknown structure.

The present invention further provides a variety of other polypeptide fusions and related multimeric proteins comprising one or more polypeptide fusions. For example, a IL-20 polypeptide can be prepared as a fusion to a dimerizing protein as disclosed in U.S. Patents Nos. 5,155,027 and 5,567,584. Preferred dimerizing proteins in this regard include immunoglobulin constant region domains. Immunoglobulin IL-20 polypeptide fusions can be expressed in genetically engineered cells. Auxiliary domains can be fused to IL-20 polypeptides to target them to specific cells, tissues, or macromolecules (e.g., collagen). For example, a IL-20 polypeptide or protein could be targeted to a predetermined cell type by fusing a polypeptide to a ligand that specifically binds to a receptor on the surface of the target cell. In this way, polypeptides and proteins can be targeted for therapeutic or diagnostic purposes. A IL-20 polypeptide can be fused to two or more moieties, such as an affinity tag for purification and a targeting domain. Polypeptide fusions can also comprise one or more cleavage sites, particularly between domains. See, Tuan et al., Connective Tissue Research 34:1 9 (1996).

Proteins according to the invention comprises derivatisation or linking to another functional molecule. The linking can be chemical coupling, genetic fusion, non-covalent association or the like, to other molecular entities such as antibodies, toxins, radioisotope, cytotoxic or cytostatic agents.

Using the methods discussed above, one of ordinary skill in the art can prepare a variety of polypeptides that are substantially identical to SEQ ID NOs: 2 or allelic variants thereof and retain the properties of the wildtype protein. As expressed and claimed herein the language, "a polypeptide as defined by SEQ ID NO: 2" includes all allelic variants and species orthologs of the polypeptide.

The protein polypeptides of the present invention, including full length proteins, protein fragments (e.g. ligand binding fragments), and fusion polypeptides can be produced in genetically engineered host cells according to conventional techniques. Suitable host cells are those cell types that can be transformed or transfected with exogenous DNA and grown in culture, and include bacteria, fungal cells, and cultured higher eukaryotic cells. Eukaryotic cells, particularly cultured cells of multicellular organisms, are preferred. Techniques for manipulating cloned DNA molecules and introducing exogenous DNA into a variety of host cells are disclosed by Sambrook et al., Molecular Cloning: A Laboratory Manual, 2nd ed.(Cold Spring Harbor Laboratory Press, Cold Spring Harbor, NY, 1989), and Ausubel et al., ibid.

It is to be recognized that according to the present invention, when a cDNA is claimed as described above, it is understood that what is claimed are both the sense strand, the anti sense strand, and the DNA as double stranded having both the sense and anti sense strand annealed together by their respective hydrogen bonds. Also claimed is the messenger RNA (mRNA) which encodes the polypeptides of the present invention, and which mRNA is encoded by the above described cDNA. A messenger RNA (mRNA) will encode a polypeptide using the same codons as those defined above, with the exception that each thymine nucleotide (T) is replaced by a uracil nucleotide (U).

To direct a IL-20 polypeptide into the secretory pathway of a host cell, a secretory signal sequence (also known as a leader sequence, prepro sequence or pre sequence) is provided in the expression vector. The secretory signal sequence may be that of the protein, or may be derived from another secreted protein (e.g., tPA) or synthesized de novo. The secretory signal sequence is joined to the IL-20 DNA sequence in the correct reading frame. Secretory signal sequences are commonly positioned 5' to the DNA sequence encoding the polypeptide of interest, although certain signal sequences may be positioned elsewhere in the DNA sequence of interest (see, e. g., Welch et al., U.S. Patent No. 5,037,743; Holland et al., U.S. Patent No. 5,143,830).

The invention also comprises chemical modifications of the IL-20 polypeptide. The chemical modification comprises covalent modifications with an organic agent capable of reacting with a selected side chain or a terminal residue. Examples of such modifications are wherein a lipophilic substituent is attached to one or more amino acid residues at a position relative to the amino acid sequence of SEQ ID NO:1 or 2 as described above. It is to be understood that an amino acid residues at the position relative to the amino acid sequence of SEQ ID NO:2 may be any amino acid residue and not only the amino acid residue naturally present at that position. In one embodiment the lipophilic substituent is attached to a lysine.

One or more of the lysines in IL-20 could be derivatives as described in the application. Relative to SEQ ID No.: 1, the lysines are located:

K₂, K₃₂, K₅₁, K₇₂, K₈₄, K₉₆, K₉₇, K₁₁₆, K₁₁₇, K₁₂₇, K₁₃₆.

Preferred embodiments are wherein the amino acid substituents are in the loop regions:

| | |
|---|---|
| Loop: | C₉VIAT₁₃ |
| Loop: | K₃₂DGNIDIRI₄₀ |
| Loop: | L₄₇QDT₅₀ |
| Loop: | Q₇₅TP₇₇ |
| Loop: | A₁₀₄HMTCHC₁₁₀ |

In preferred embodiments the lysines are in the loop region, such as: K2, K32

In other preferred embodiments, additional lysines are substituted, inserted into the sequence or added at the N-terminal or C-terminal, and then optionally derivatised.

Preferred regions of insertions are where the overall activity of the protein is not adversely affected. Preferred regions are the loop region.

Deletion analogues of the IL-20 peptide includes, in the N-terminal:
deletions of the signal sequence of parts of the signal sequence: MKASS-LAFSLLSAAFYLLWTPSTG
   and further deletions in the N-terminal:
   - deletions of the L
   - deletion of LK
   - deletion of LKT
   - deletion of LKTL
   - deletion of LKTLN
   - deletion of LKTLNL
   - deletion of LKTLNLG
   - deletion of LKTLNLGS
   - deletion of LKTLNLGSC
   - deletion of LKTLNLGSCV
   - deletion of LKTLNLGSCVI
   - deletion of LKTLNLGSCVIA
   - deletion of LKTLNLGSCVIAT
   - deletion of LKTLNLGSCVIATN

Deletion analogues of the IL-20 peptide includes, in the C-terminal:
- deletion of ELDILLQWMEETE
- deletion of LDILLQWMEETE
- deletion of DILLQWMEETE
- deletion of ILLQWMEETE
- deletion of LLQWMEETE
- deletion of LQWMEETE
- deletion of QWMEETE
- deletion of WMEETE
- deletion of MEETE
- deletion of EETE
- deletion of ETE
- deletion of TE
- deletion of E

N-terminal and C-terminal truncations may occur simultaneously.

The term "lipophilic substituent" is characterised by comprising 4-40 carbon atoms and having a solubility in water at 20°C in the range from about 0.1 mg/100 ml water to about 250 mg/100 ml water, such as in the range from about 0.3 mg/100 ml water to about 75 mg/100 ml water. For instance, octanoic acid (C8) has a solubility in water at 20°C of 68 mg/100 ml, decanoic acid (C10) has a solubility in water at 20°C of 15 mg/100 ml, and octadecanoic acid (C18) has a solubility in water at 20°C of 0.3 mg/100 ml.

To obtain a satisfactory protracted profile of action of the IL-20 derivative, the lipophilic substituent attached to the IL-20 moiety, as an example comprises 4-40 carbon atoms, such as 8-25 carbon atoms. The lipophilic substituent may be attached to an amino group of the I L-20 moiety by means of a carboxyl group of the lipophilic substituent which forms an amide bond with an amino group of the amino acid to which it is attached. As an alternative, the lipophilic substituent may be attached to said amino acid in such a way that an amino group of the lipophilic substituent forms an amide bond with a carboxyl group of the amino acid. As a further option, the lipophililic substituent may be linked to the IL-20 moiety via an ester bond. Formally, the ester can be formed either by reaction between a carboxyl group of the IL-20 moiety and a hydroxyl group of the substituent-to-be or by reaction between a hydroxyl group of the IL-20 moiety and a carboxyl group of the substituent-to-be. As a further alternative, the lipophilic substituent can be an alkyl group which is introduced into a primary amino group of the IL-20 moiety.

In one embodiment of the invention the IL-20 derivative only has one lipophilic substituent attached to the IL-20 peptide.

In one embodiment of the invention the lipophilic substituent comprises from 4 to 40 carbon atoms.

In one embodiment of the invention the lipophilic substituent comprises from 8 to 25 carbon atoms.

In one embodiment of the invention the lipophilic substituent comprises from 12 to 20 carbon atoms.

In one embodiment of the invention the lipophilic substituent is attached to an amino acid residue in such a way that a carboxyl group of the lipophilic substituent forms an amide bond with an amino group of the amino acid residue.

In one embodiment of the invention the lipophilic substituent is attached to a Lys residue. One or more of the lysines in IL-20 could be derivatives as described in the application. Relative to SEQ ID No.: 1, the lysines are located:

K₂, K₃₂, K₅₁, K₇₂, K₈₄, K₉₆, K₉₇, K₁₁₆, K₁₁₇, K₁₂₇, K₁₃₆

Preferred embodiments are wherein the amino acid substituents are in the loop regions:

| | |
|---|---|
| Loop: | C₉VIAT₁₃ |
| Loop: | K₃₂DGNIDIRI₄₀ |
| Loop: | L₄₇QDT₅₀ |
| Loop: | Q₇₅TP₇₇ |
| Loop: | A₁₀₄HMTCHC₁₁₀ |

In preferred embodiments the lysines are in the loop region, such as: K2, K32

In other preferred embodiments, additional lysines are substituted, inserted into the sequence or added at the N-terminal or C-terminal, and then optionally derivatised.

Preferred regions of insertions are where the overall activity of the protein is not adversely affected. Preferred regions are the loop region.

In one embodiment of the invention the lipophilic substituent is attached to an amino acid residue in such a way that an amino group of the lipophilic substituent forms an amide bond with a carboxyl group of the amino acid residue.

In one embodiment of the invention the lipophilic substituent is attached to the IL-20 peptide by means of a spacer.

In one embodiment of the invention the spacer is an unbranched alkane α,ω-dicarboxylic acid group having from 1 to 7 methylene groups, such as two methylene groups which spacer forms a bridge between an amino group of the IL-20 peptide and an amino group of the lipophilic substituent.

In one embodiment of the invention the spacer is an amino acid residue except a Cys residue, or a dipeptide. Examples of suitable spacers includes β-alanine, gamma-aminobutyric acid (GABA), γ-glutamic acid, succinic acid, Lys, Glu or Asp, or a dipeptide such as Gly-Lys. When the spacer is succinic acid, one carboxyl group thereof may form an amide bond with an amino group of the amino acid residue, and the other carboxyl group thereof may form an amide bond with an amino group of the lipophilic substituent. When the spacer is Lys, Glu or Asp, the carboxyl group thereof may form an amide bond with an amino group of the amino acid residue, and the amino group thereof may form an amide bond with a carboxyl group of the lipophilic substituent. When Lys is used as the spacer, a further spacer may in some instances be inserted between the ε-amino group of Lys and the lipophilic substituent. In one embodiment, such a further spacer is succinic acid which forms an amide bond with the ε-amino group of Lys and with an amino group present in the lipophilic substituent. In another embodiment such a further spacer is Glu or Asp which forms an amide bond with the ε-amino group of Lys and another amide bond with a carboxyl group present in the lipophilic substituent, that is, the lipophilic substituent is a N^{ε}-acylated lysine residue.

In one embodiment of the invention the spacer is selected from the list consisting of β-alanine, gamma-aminobutyric acid (GABA), γ-glutamic acid, Lys, Asp, Glu, a dipeptide con-taining Asp, a dipeptide containing Glu, or a dipeptide containing Lys. In one embodiment of the invention the spacer is □-alanine. In one embodiment of the invention the spacer is gamma-aminobutyric acid (GABA). In one embodiment of the invention the spacer is γ-glutamic acid.

In one embodiment of the invention a carboxyl group of the parent IL-20 peptide forms an amide bond with an amino group of a spacer, and the carboxyl group of the amino acid or dipeptide spacer forms an amide bond with an amino group of the lipophilic substituent.

In one embodiment of the invention an amino group of the parent IL-20 peptide forms an amide bond with a carboxylic group of a spacer, and an amino group of the spacer forms an amide bond with a carboxyl group of the lipophilic substituent.

In one embodiment of the invention the lipophilic substituent comprises a partially or completely hydrogenated cyclopentanophenathrene skeleton.

In one embodiment of the invention the lipophilic substituent is an straight-chain or branched alkyl group. In one embodiment of the invention the lipophilic substituent is the acyl group of a straight-chain or branched fatty acid.

In one embodiment of the invention the acyl group of a lipophilic substituent is selected from the group comprising CH₃(CH₂)ₙCO-, wherein n is 4 to 38, such as CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- and CH₃(CH₂)₂₂CO-.

In one embodiment of the invention the lipophilic substituent is an acyl group of a straight-chain or branched alkane α,ω-dicarboxylic acid.

In one embodiment of the invention the acyl group of the lipophilic substituent is selected from the group comprising HOOC(CH₂)ₘCO-, wherein m is 4 to 38, such as HOOC(CH₂)₁₄CO-, HOOC(CH₂)₁₆CO-, HOOC(CH₂)₁₈CO-, HOOC(CH₂)₂₀CO- and HOOC(CH₂)₂₂CO-.

In one embodiment of the invention the lipophilic substituent is a group of the formula CH₃(CH₂)p((CH₂)qCOOH)CHNH-CO(CH₂)₂CO-, wherein p and q are integers and p+q is an integer of from 8 to 40, such as from 12 to 35.

In one embodiment of the invention the lipophlic substituent is a group of the formula CH₃(CH₂)ᵣCO-NHCH(COOH)(CH₂)₂CO-, wherein r is an integer of from 10 to 24. In one embodiment of the invention the lipophilic substituent is a group of the formula CH₃(CH₂)ₛCO-NHCH((CH₂)₂COOH)CO-, wherein s is an integer of from 8 to 24.

In one embodiment of the invention the lipophilic substituent is a group of the formula COOH(CH₂)ₜCO- wherein t is an integer of from 8 to 24.

In one embodiment of the invention the lipophilic substituent is a group of the formula -NHCH(COOH)(CH₂)₄NH-CO(CH₂)ᵤCH₃, wherein u is an integer of from 8 to 18.

In one embodiment of the invention the lipophilic substituent is a group of the formula -NHCH(COOH)(CH₂)₄NH-COCH((CH₂)₂COOH)NH-CO(CH₂)_{w}CH₃, wherein w is an integer of from 10 to 16.

In one embodiment of the invention the lipophilic substituent is a group of the formula -NHCH(COOH)(CH₂)₄NH-CO(CH₂)₂CH(COOH)NH-CO(CH₂)ₓCH₃, wherein x is an integer of from 10 to 16.

In one embodiment of the invention the lipophilic substituent is a group of the formula -NHCH(COOH)(CH₂)₄NH-CO(CH₂)₂CH(COOH)NHCO(CH₂)_{y}CH₃, wherein y is zero or an integer of from 1 to 22.

In one embodiment of the invention the lipophilic substituent is N-Lithocholoyl.

In one embodiment of the invention the lipophilic substituent is N-Choloyl.

In one embodiment of the invention the IL-20 derivative has one lipophilic substituent. In one embodiment of the invention the IL-20 derivative has two lipophilic substituents. In one embodiment of the invention the IL-20 derivative has three lipophilic substituents. In one embodiment of the invention the IL-20 derivative has four lipophilic substituents.

The methods of the present invention also contemplate using chemically modified IL-20 compositions, in which a IL-20 polypeptide is linked with a polymer. Illustrative IL-20 polypeptides are soluble polypeptides that lack a functional transmembrane domain, such as a mature IL-20 polypeptide. Typically, the polymer is water soluble so that the IL-20 conjugate does not precipitate in an aqueous environment, such as a physiological environment. An example of a suitable polymer is one that has been modified to have a single reactive group, such as an active ester for acylation, or an aldehyde for alkylation. In this way, the degree of polymerization can be controlled. An example of a reactive aldehyde is polyethylene glycol propionaldehyde, or mono-(C1-C10) alkoxy, or aryloxy derivatives thereof (see, for example, Harris, et al., U.S. Patent No. 5,252,714). The polymer may be branched or unbranched. Moreover, a mixture of polymers can be used to produce IL-20 conjugates.

IL-20 conjugates used for therapy can comprise pharmaceutically acceptable water-soluble polymer moieties. Suitable water-soluble polymers include polyethylene glycol (PEG), monomethoxy-PEG, mono-(C₁-C₁₀)alkoxy-PEG, aryloxy-PEG, poly-(N-vinyl pyrrolidone)PEG, tresyl monomethoxy PEG, PEG propionaldehyde, bis-succinimidyl carbonate PEG, propylene glycol homopolymers, a polypropylene oxide/ethylene oxide co-polymer, polyoxyethylated polyols (*e.g.*, glycerol), polyvinyl alcohol, dextran, cellulose, or other carbohydrate-based polymers. Suitable PEG may have a molecular weight from about 600 to about 60,000, including, for example, 5,000, 12,000, 20,000 and 25,000. A IL-20 conjugate can also comprise a mixture of such water-soluble polymers.

Percentage sequence identity between two amino acid sequences is determined by a Needelman-Wunsch alignment, useful for both protein and DNA alignments. For protein alignments the default scoring matrix used is BLOSUM50, and the penalty for the first residue in a gap is -12, while the penalty for additional residues in a gap is -2. The alignment may be made with the Align software from the FASTA package version v20u6 (W.R. Pearson and D.J. Lipman (1988), "Improved Tools for Biological Sequence Analysis", PNAS 85:2444-2448; and W.R. Pearson (1990) "Rapid and Sensitive Sequence Comparison with FASTP and FASTA", Methods in Enzymology, 183:63-98).

In one embodiment the polypeptide used in the present invention is an isolated polypeptide. In another embodiment the polynucleotide used in the present invention is an isolated polynucleotide.

It is preferred to purify the polypeptides of the present invention to :>80% purity, more preferably to >90% purity, even more preferably >95% purity, and particularly preferred is a pharmaceutically pure state, that is greater than 99.9% pure with respect to contaminating macromolecules, particularly other proteins and nucleic acids, and free of infectious and pyrogenic agents. Preferably, a purified polypeptide is substantially free of other polypeptides, particularly other polypeptides of animal origin.

The medium used to culture the cells may be any conventional medium suitable for growing the host cells, such as minimal or complex media containing appropriate supplements. Suitable media are available from commercial suppliers or may be prepared according to published recipes (e.g. in catalogues of the American Type Culture Collection). The media are prepared using procedures known in the art (see, e.g., references for bacteria and yeast; Bennett, J.W. and LaSure, L., editors, More Gene Manipulations in Fungi, Academic Press, CA, 1991).

If the polypeptide is secreted into the nutrient medium, the polypeptide can be recovered directly from the medium. If the polypeptide is not secreted, it can be recovered from cell lysates. The polypeptide may be recovered from the culture medium by conventional procedures including separating the host cells from the medium by centrifugation or filtration.

The polypeptides may be detected using methods known in the art that are specific for the polypeptides. These detection methods may include use of specific antibodies, formation of an enzyme product, or disappearance of an enzyme substrate. For example, an enzyme assay may be used to determine the activity of the polypeptide.

The resulting polypeptide may be recovered by methods known in the art. For example, the polypeptide may be recovered from the nutrient medium by conventional procedures including, but not limited to, centrifugation, filtration, extraction, spray-drying, evaporation, or precipitation.

The polypeptides of the present invention may be purified by a variety of procedures known in the art including, but not limited to, chromatography (e.g., ion exchange, affinity, hydro-phobic, chromatofocusing, and size exclusion), electrophoretic procedures (e.g., preparative isoelectric focusing), differential solubility (e.g., ammonium sulfate precipitation), SDS-PAGE, or extraction (see, e.g., Protein Purification, J.-C. Janson and Lars Ryden, editors, VCH Publishers, New York, 1989).

In the production methods of the present invention, the cells are cultivated in a nutrient medium suitable for production of the polypeptide using methods known in the art. For example, the cell may be cultivated by shake flask cultivation, small-scale or large-scale fermentation (including continuous, batch, fed-batch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art.

IL-20 antagonists are as well soluble receptors, antibodies, peptides or small molecules. Antagonists are acting on the IL-20 receptor complex or on IL-20 ligand, but they function as antagonists on the receptor, or they bind to either IL-20 or the receptor and thereby preventing IL-20 from acting on the receptor. In an aspect of the invention the antagonist is an anti-body against IL-20. In another aspect of the invention the antagonist is a soluble receptor. Soluble receptors are described for example in WO 01/46261, WO 01/46232, WO 02/22153 or WO 02/72607 (ZymoGenetics).

IL-20 may be administered as an agonist in combination therapy with other neovascularisa-tion promoting compounds, such as VEGF, acidic or basic FGF, PDGF, MMP's

IL-20 antagonist may be administered as a combination therapy with other neovascularisation reducing compounds. Examples of such compounds such as TNF and IFN-alfa, an anti-body capable of inhibiting or neutralising the angiogenic activity of acidic or basic fibroblast growth factor (FGF), antagonists of VEGF or other endothelial cell proliferating inhibitors, or hepatocyte growth factor (HGH), an antibody capable of inhibiting or neutralising the coagulant activities of tissue factor, protein C, or protein S (WO 91/01753) or one or more conventional therapeutic agents such as alkylating agents, folic acid antagonists, antimetabolites of nucleic acid metabolism, antibiotics, pyrimidine analogues, 5-fluorouracil, purine nucleosides, amines, amino acids, triazol nucleosides, or corticosteroids. Such other agents may be pre-sent in the composition being administered or may be administered separately. Also, the treatment may be further combined with radiological treatment such as irradiation or administration of radioactive substances.

In other embodiments of the invention, IL-20 antagonist may be administered in combination with inhibitors of extra cellular matrix dissolving agents such as inhibitors of MMP's (MMP=metalloproteinase), tubulin-binding agents, calcium-flux inhibitors, COX-2 inhibitors, upregulators of IFN-gamma and IP-10, inhibitors of integrin binding and survival signaling or sodium-hydrogen ion inhibitors.

In other embodiments of the invention, vascularisation of tumors are attacked in combination therapy such as by administering antagonists of I L-20 to prevent neovascularisation, followed by, or concurrent administration of, for example TNF alone or in combination with further agents such as heregulin, anti-heregulin antibody, D-factor, interleukin-1, Interleukin-2, granulocyte-macrophage colony stimulating factor (GM-CSF) or agents the promote microvascular coagulation such as anti protein C antibody, anti-protein S antibody, or C4b binding protein, heat or irradiation. The treatment impact on the tumor can be monitored by conventional matrix screening. In other embodiments, a FGF or platelet-derived growth factor (PDGF) antagonist, such as an FGF or an anti-PDGF neutralising antibody, is administered to the patient in conjunction with the IL-20 antagonist. Treatments may be suspended during period of wound healing or desirable neovascularisation.

In an aspect of the invention antagonists of IL-20 for the prevention of neovascularisation in tumor cells - for prevention of metastasis, are use in combination with and one or more of the therapies mentioned below.
I. Agents that induce tumor cell death or death of virus-infected cells
   a) conventional chemotherapy
   b) radiation therapy
   c) monoclonal antibodies
   d) cell cycle control/apoptosis regulators
   e) growth factor and signal transduction modulators
   f) inhibitors of tumor vascularisation (angiogenesis inhibitors, antiangiogenesis drugs)
   g) Viral targeting (the use of a recombinant virus to destroy tumor cells)
   h) anti-viral agents
   i) Hormonal agents
II. Agents that enhance the immune response against tumor cells or virus-infected cells
   j) immune system activators
   k) immune system inhibitors (e.g. agents that inhibit immune signals down-regulating the immune response), including anti-anergic agents
   l) therapeutic vaccines
III. Agents that interfere with tumor growth, metastasis or spread of virus-infected cells
   m) integrins, cell adhesion molecules modulators
   n) anti-metastatics
   o) endothelial cell modulators
IV. Internal vaccination.
V. Tissue factor antagonist and other factors influencing the coagulation cascade
   p) anti Factor Xa, anti Factor IIa inhibitors, anti-fibrinogenic agents
   q) pentasaccharides etc.
VI. Immunosuppressive / immunomodulatory agents
   r) agents with influence on T-lymphocyte homing e.g. FTY-720
   s) calcineurin inhibitors
   t) TOR inhibitors

For the use in diagnosis and detection of neovascularisation activity in an animal, the probe or antibody is labelled with a moiety producing a detectable signal, ie a radionucleide, enzyme, contract agent or fluorophore. Labelled second antibodies or other labelled secondary agents can also be employed. The probe or antibody can be administered to the patient and detected in vivo by conventional scanning methods or it can be used in vitro in biopsies and tissue samples.

### PHARMACEUTICAL COMPOSITIONS

The present invention also relates to pharmaceutical compositions comprising, as an active ingredient, at least one of the compounds (polypeptide, antibody, soluble receptor, small molecule or polynucleotide) of the present invention or a pharmaceutically acceptable salt thereof and, usually, such compositions also contain a pharmaceutically acceptable carrier, surfactant or diluent. The pharmaceutical compositions of the invention can also comprise combinations with other compounds as described.

Pharmaceutical compositions comprising a compound of the present invention may be prepared by conventional techniques, e.g. as described in Remington: The Science and Practise of Pharmacy, 19th Ed., 1995. The compositions may appear in conventional forms, for example capsules, tablets, aerosols, solutions or suspensions.

The pharmaceutical compositions may be specifically formulated for administration by any suitable route such as the oral, rectal, nasal, pulmonary, topical (including buccal and sublingual), transdermal, intracisternal, intraperitoneal, vaginal and parenteral (including subcutaneous, intramuscular, intrathecal, intravenous and intradermal) route. It will be appreciated that the preferred route will depend on the general condition and age of the subject to be treated, the nature of the condition to be treated and the active ingredient chosen. The route of administration may be any route, which effectively transports the active compound to the appropriate or desired site of action.

Pharmaceutical compositions for oral administration include solid dosage forms such as hard or soft capsules, tablets, troches, dragees, pills, lozenges, powders and granules. Where appropriate, they can be prepared with coatings such as enteric coatings or they can be formulated so as to provide controlled release of the active ingredient such as sustained or prolonged release according to methods well known in the art.

Liquid dosage forms for oral administration include solutions, emulsions, aqueous or oily suspensions, syrups and elixirs.

Pharmaceutical compositions for parenteral administration include sterile aqueous and non-aqueous injectable solutions, dispersions, suspensions or emulsions as well as sterile powders to be reconstituted in sterile injectable solutions or dispersions prior to use. Depot injectable formulations are also contemplated as being within the scope of the present invention.

Other suitable administration forms include suppositories, sprays, ointments, cremes, gels, inhalants, dermal patches, implants etc.

A typical oral dosage is in the range of from about 0.001 to about 100 mg/kg body weight per day, such as from about 0.01 to about 50 mg/kg body weight per day, for example from about 0.05 to about 10 mg/kg body weight per day administered in one or more dosages such as 1 to 3 dosages. The exact dosage will depend upon the nature of the IL-20 or the I L-20 mimetic, together with the combination agent chosen, the frequency and mode of administration, the sex, age, weight and general condition of the subject treated, the nature and severity of the condition treated and any concomitant diseases to be treated and other factors evident to those skilled in the art.

The formulations may conveniently be presented in unit dosage form by methods known to those skilled in the art. A typical unit dosage form for oral administration one or more times per day such as 1 to 3 times per day may contain from 0.05 to about 1000 mg, for example from about 0.1 to about 500 mg, such as from about 0.5 mg to about 200 mg.

For parenteral routes such as intravenous, intrathecal, intramuscular and similar administration, typically doses are in the order of about half the dose employed for oral administration.

Salts of polypeptides or small molecules are especially relevant when the compounds is in solid or crystalline form

For parenteral administration, solutions of the compounds of the invention, optionally together with the combination agent in sterile aqueous solution, aqueous propylene glycol or sesame or peanut oil may be employed. Such aqueous solutions should be suitably buffered if necessary and the liquid diluent first rendered isotonic with sufficient saline or glucose. The aqueous solutions are particularly suitable for intravenous, intramuscular, subcutaneous and intraperitoneal administration. The sterile aqueous media employed are all readily available by standard techniques known to those skilled in the art.

Suitable pharmaceutical carriers include inert solid diluents or fillers, sterile aqueous solution and various organic solvents. Examples of solid carriers are lactose, terra alba, sucrose, cyclodextrin, talc, gelatine, agar, pectin, acacia, magnesium stearate, stearic acid and lower alkyl ethers of cellulose. Examples of liquid carriers are syrup, peanut oil, olive oil, phospholipids, fatty acids, fatty acid amines, polyoxyethylene and water. Similarly, the carrier or diluent may include any sustained release material known in the art, such as glyceryl monostearate or glyceryl distearate, alone or mixed with a wax.

The pharmaceutical compositions formed by combining a compound of the invention and the pharmaceutically acceptable carriers are then readily administered in a variety of dosage forms suitable for the disclosed routes of administration. The formulations may conveniently be presented in unit dosage form by methods known in the art of pharmacy.

For nasal administration, the preparation may contain a compound of the invention dissolved or suspended in a liquid carrier, in particular an aqueous carrier, for aerosol application. The carrier may contain additives such as solubilizing agents, e.g. propylene glycol, surfactants, absorption enhancers such as lecithin (phosphatidylcholine) or cyclodextrin, or preservatives such as parabenes.

Formulations of a compound of the invention suitable for oral administration may be presented as discrete units such as capsules or tablets, each containing a predetermined amount of the active ingredient, and which may include a suitable excipient. Furthermore, the orally available formulations may be in the form of a powder or granules, a solution or suspension in an aqueous or non-aqueous liquid, or an oil-in-water or water-in-oil liquid emulsion.

Compositions intended for oral use may be prepared according to any known method, and such compositions may contain one or more agents selected from the group consisting of sweetening agents, flavouring agents, colouring agents, and preserving agents in order to provide pharmaceutically elegant and palatable preparations. Tablets may contain the active ingredient in admixture with non-toxic pharmaceutically-acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example corn starch or alginic acid; binding agents, for example, starch, gelatine or acacia; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in U.S. Patent Nos. 4,356,108; 4,166,452; and 4,265,874, incorporated herein by reference, to form osmotic therapeutic tablets for controlled release.

Formulations for oral use may also be presented as hard gelatine capsules where the active ingredient is mixed with an inert solid diluent, for example, calcium carbonate, calcium phosphate or kaolin, or a soft gelatine capsules wherein the active ingredient is mixed with water or an oil medium, for example peanut oil, liquid paraffin, or olive oil.

Aqueous suspensions may contain a compound of the invention in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide such as lecithin, or condensation products of an alkylene oxide with fatty acids, for example polyoxyethylene stearate, or condensation products of ethylene oxide with long chain aliphatic alcohols, for example, heptadecaethyl-eneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as a liquid paraffin. The oily suspensions may contain a thickening agent, for example beeswax, hard paraffin or cetyl alcohol. Sweetening agents such as those set forth above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water provide the active compound in admixture with a dispersing or wetting agent, suspending agent and one or more preservatives. Suitable dispersing or wetting agents and suspending agents are exemplified by those already mentioned above. Additional excipients, for example, sweetening, flavouring, and colouring agents may also be present.

The pharmaceutical compositions of a compound of the invention may also be in the form of oil-in-water emulsions. The oily phase may be a vegetable oil, for example, olive oil or arachis oil, or a mineral oil, for example a liquid paraffin, or a mixture thereof. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of said partial esters with ethylene oxide, for example polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a demulcent, preservatives and flavouring and colouring agents. The pharmaceutical compositions may be in the form of a sterile injectible aqueous or oleaginous suspension. This suspension may be formulated according to the known methods using suitable dispersing or wetting agents and suspending agents described above. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution, and isotonic sodium chloride solution. In addition, sterile, fixed oils are conveniently employed as solvent or suspending medium. For this purpose, any bland fixed oil may be employed using synthetic mono- or diglycerides. In addition, fatty acids such as oleic acid find use in the preparation of injectables.

The compositions may also be in the form of suppositories for rectal administration of the compounds of the invention. These compositions can be prepared by mixing the drug with a suitable non-irritating excipient which is solid at ordinary temperatures but liquid at the rectal temperature and will thus melt in the rectum to release the drug. Such materials include cocoa butter and polyethylene glycols, for example.

For topical use, creams, ointments, jellies, solutions of suspensions, etc., containing the compounds of the invention are contemplated. For the purpose of this application, topical applications shall include mouth washes and gargles.

A compound of the invention may also be administered in the form of liposome delivery systems, such as small unilamellar vesicles, large unilamellar vesicles, and multilamellar vesicles. Liposomes may be formed from a variety of phospholipids, such as cholesterol, stearylamine, or phosphatidylcholines.

In addition, some of the compounds of the invention may form solvates with water or common organic solvents. Such solvates are also encompassed within the scope of the invention.

If a solid carrier is used for oral administration, the preparation may be tabletted, placed in a hard gelatine capsule in powder or pellet form or it can be in the form of a troche or lozenge. The amount of solid carrier will vary widely but will usually be from about 25 mg to about 1 g. If a liquid carrier is used, the preparation may be in the form of a syrup, emulsion, soft gelatine capsule or sterile injectable liquid such as an aqueous or non aqueous liquid suspension or solution.

A compound of the invention may be administered to a mammal, especially a human, in need of such treatment. Such mammals include also animals, both domestic animals, e.g. household pets, and non-domestic animals such as wildlife.

Pharmaceutical compositions containing a compound according to the invention may be administered one or more times per day or week, conveniently administered at meal-times. An effective amount of such a pharmaceutical composition is the amount that provides a clinically significant effect. Such amounts will depend, in part, on the particular condition to be treated, age, weight, and general health of the patient, and other factors evident to those skilled in the art.

In one embodiment the invention relates to a pharmaceutical composition of the invention comprising an amount of a compound of the invention effective to promote neovascularisation.

In another embodiment the invention relates to a pharmaceutical composition of the invention comprising an amount of a compound of the invention effective to inhibit neovascularisation.

A convenient daily dosage can be in the range from 1-1000 microgram/kg/day. In another embodiment from 5-500 microgram/kg/day. If the body weight of the subject changes during treatment, the dose of the compound might have to be adjusted accordingly.

The invention provides in a particular embodiment the following:

Another object of the present invention is to provide a pharmaceutical formulation comprising I L-20, analogues or derivatives thereof, or optionally together with any other compound mentioned in the present application which is present in a concentration from 0.1 mg/ml to 100 mg/ml, and wherein said formulation has a pH from 2.0 to 10.0. The formulation may further comprise a buffer system, preservative(s), tonicity agent(s), chelating agent(s), stabilizers and surfactants. In one embodiment of the invention the pharmaceutical formulation is an aqueous formulation, i.e. formulation comprising water. Such formulation is typically a solution or a suspension. In a further embodiment of the invention the pharmaceutical formulation is an aqueous solution. The term "aqueous formulation" is defined as a formulation comprising at least 50 %w/w water. Likewise, the term "aqueous solution" is defined as a solution comprising at least 50 %w/w water, and the term "aqueous suspension" is defined as a suspension comprising at least 50 %w/w water.

In another embodiment the pharmaceutical formulation is a freeze-dried formulation, whereto the physician or the patient adds solvents and/or diluents prior to use.

In another embodiment the pharmaceutical formulation is a dried formulation (e.g. freeze-dried or spray-dried) ready for use without any prior dissolution.

In a further aspect the invention relates to a pharmaceutical formulation comprising an aqueous solution of IL-20 or any other compound as mentioned above and a buffer, wherein said compound is present in a concentration from 0.1 mg/ml or as mentioned above, preferably from 0.5 mg/ml - 50 mg/ml and wherein said formulation has a pH from about 2.0 to about 10.0. Preferred pH is from 3.0 to about 8.0. Particular preferred range is from 4.0-6.0, such as for example the ranges 4.0-4.5, 4.5 -5.0, 5.0-5.5 and 5.5-6.0.

In another embodiment of the invention the pH of the formulation is selected from the list consisting of 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, and 10.0.

In a further embodiment of the invention the buffer is selected from the group consisting of sodium acetate, sodium carbonate, citrate, glycylglycine, histidine, glycine, lysine, arginine, sodium dihydrogen phosphate, disodium hydrogen phosphate, sodium phosphate, and tris(hydroxymethyl)-aminomethan, bicine, tricine, malic acid, succinate, maleic acid, fumaric acid, tartaric acid, aspartic acid or mixtures thereof. Each one of these specific buffers constitutes an alternative embodiment of the invention.

In a further embodiment of the invention the formulation further comprises a pharmaceutically acceptable antimicrobial preservative. In a further embodiment of the invention the preservative is selected from the group consisting of phenol, o-cresol, m-cresol, p-cresol, methyl p-hydroxybenzoate, propyl p-hydroxybenzoate, 2-phenoxyethanol, butyl p-hydroxybenzoate, 2-phenylethanol, benzyl alcohol, chlorobutanol, and thiomersal, bronopol, benzoic acid, imidurea, chlorohexidine, sodium dehydroacetate, chlorocresol, ethyl p-hydroxybenzoate, benzethonium chloride, chlorphenesine (3p-chlorphenoxypropane-1,2-diol) or mixtures thereof. In a further embodiment of the invention the preservative is present in a concentration from 0.1 mg/ml to 20 mg/ml. In a further embodiment of the invention the preservative is present in a concentration from 0.1 mg/ml to 5 mg/ml. In a further embodiment of the invention the preservative is present in a concentration from 5 mg/ml to 10 mg/ml. In a further embodiment of the invention the preservative is present in a concentration from 10 mg/ml to 20 mg/ml. Each one of these specific preservatives constitutes an alternative embodiment of the invention. The use of a preservative in pharmaceutical compositions is well-known to the skilled person. For convenience reference is made to Remington: The Science and Practice of Pharmacy, 19th edition, 1995.

In a further embodiment of the invention the formulation further comprises an isotonic agent. In a further embodiment of the invention the isotonic agent is selected from the group consisting of a salt (e.g. sodium chloride), a sugar or sugar alcohol, an amino acid (e.g. L-glycine, L-histidine, arginine, lysine, isoleucine, aspartic acid, tryptophan, threonine), an alditol (e.g. glycerol (glycerine), 1,2-propanediol (propyleneglycol), 1,3-propanediol, 1,3-butanediol) polyethyleneglycol (e.g. PEG400), or mixtures thereof. Any sugar such as mono-, di-, or polysaccharides, or water-soluble glucans, including for example fructose, glucose, mannose, sorbose, xylose, maltose, lactose, sucrose, trehalose, dextran, pullulan, dextrin, cyclodextrin, soluble starch, hydroxyethyl starch and carboxymethylcellulose-Na may be used. In one embodiment the sugar additive is sucrose. Sugar alcohol is defined as a C4-C8 hydrocarbon having at least one --OH group and includes, for example, mannitol, sorbitol, inositol, galactitol, dulcitol, xylitol, and arabitol. In one embodiment the sugar alcohol additive is mannitol. The sugars or sugar alcohols mentioned above may be used individually or in combination. There is no fixed limit to the amount used, as long as the sugar or sugar alcohol is soluble in the liquid preparation and does not adversely effect the stabilizing effects achieved using the methods of the invention. In one embodiment, the sugar or sugar alcohol concentration is between about 1 mg/ml and about 150 mg/ml. In a further embodiment of the invention the isotonic agent is present in a concentration from 1 mg/ml to 50 mg/ml. In a further embodiment of the invention the isotonic agent is present in a concentration from 1 mg/ml to 7 mg/ml. In a further embodiment of the invention the isotonic agent is present in a concentration from 8 mg/ml to 24 mg/ml. In a further embodiment of the invention the isotonic agent is present in a concentration from 25 mg/ml to 50 mg/ml. Each one of these specific isotonic agents constitutes an alternative embodiment of the invention. The use of an isotonic agent in pharmaceutical compositions is well-known to the skilled person. For convenience reference is made to Remington: The Science and Practice of Pharmacy, 19th edition, 1995.

In a further embodiment of the invention the formulation further comprises a chelating agent. In a further embodiment of the invention the chelating agent is selected from salts of ethylenediaminetetraacetic acid (EDTA), citric acid, and aspartic acid, and mixtures thereof. In a further embodiment of the invention the chelating agent is present in a concentration from 0.1 mg/ml to 5mg/ml. In a further embodiment of the invention the chelating agent is present in a concentration from 0.1 mg/ml to 2mg/ml. In a further embodiment of the invention the chelating agent is present in a concentration from 2mg/ml to 5mg/ml. Each one of these specific chelating agents constitutes an alternative embodiment of the invention. The use of a chelating agent in pharmaceutical compositions is well-known to the skilled person. For convenience reference is made to Remington: The Science and Practice of Pharmacy, 19th edition, 1995.

In a further embodiment of the invention the formulation further comprises a stabilizer. The use of a stabilizer in pharmaceutical compositions is well-known to the skilled person. For convenience reference is made to Remington: The Science and Practice of Pharmacy, 19th edition, 1995.

More particularly, compositions of the invention are stabilized liquid pharmaceutical compositions whose therapeutically active components include a polypeptide that possibly exhibits aggregate formation during storage in liquid pharmaceutical formulations. By "aggregate formation" is intended a physical interaction between the polypeptide molecules that results in formation of oligomers, which may remain soluble, or large visible aggregates that precipitate from the solution. By "during storage" is intended a liquid pharmaceutical composition or formulation once prepared, is not immediately administered to a subject. Rather, following preparation, it is packaged for storage, either in a liquid form, in a frozen state, or in a dried form for later reconstitution into a liquid form or other form suitable for administration to a subject. By "dried form" is intended the liquid pharmaceutical composition or formulation is dried either by freeze drying (i.e., lyophilization; see, for example, Williams and Polli (1984) J. Parenteral Sci. Technol. 38:48-59), spray drying (see Masters (1991) in Spray-Drying Handbook (5th ed; Longman Scientific and Technical, Essez, U.K.), pp. 491-676; Broadhead et al. (1992) Drug Devel. Ind. Pharm. 18:1169-1206; and Mumenthaler et al. (1994) Pharm. Res. 11:12-20), or air drying (Carpenter and Crowe (1988) Cryobiology 25:459-470; and Roser (1991) Biopharm. 4:47-53). Aggregate formation by a polypeptide during storage of a liquid pharmaceutical composition can adversely affect biological activity of that polypeptide, resulting in loss of therapeutic efficacy of the pharmaceutical composition. Furthermore, aggregate formation may cause other problems such as blockage of tubing, membranes, or pumps when the polypeptide-containing pharmaceutical composition is administered using an infusion system.

The pharmaceutical compositions of the invention may further comprise an amount of an amino acid base sufficient to decrease aggregate formation by the polypeptide during storage of the composition. By "amino acid base" is intended an amino acid or a combination of amino acids, where any given amino acid is present either in its free base form or in its salt form. Where a combination of amino acids is used, all of the amino acids may be present in their free base forms, all may be present in their salt forms, or some may be present in their free base forms while others are present in their salt forms. In one embodiment, amino acids to use in preparing the compositions of the invention are those carrying a charged side chain, such as arginine, lysine, aspartic acid, and glutamic acid. Any stereoisomer (i.e., L, D, or DL isomer) of a particular amino acid (e.g. glycine, methionine, histidine, imidazole, arginine, lysine, isoleucine, aspartic acid, tryptophan, threonine and mixtures thereof) or combinations of these stereoisomers, may be present in the pharmaceutical compositions of the invention so long as the particular amino acid is present either in its free base form or its salt form. In one embodiment the L-stereoisomer is used. Compositions of the invention may also be formulated with analogues of these amino acids. By "amino acid analogue" is intended a derivative of the naturally occurring amino acid that brings about the desired effect of decreasing aggregate formation by the polypeptide during storage of the liquid pharmaceutical compositions of the invention. Suitable arginine analogues include, for example, aminoguanidine, ornithine and N-monoethyl L-arginine, suitable methionine analogues include ethionine and buthionine and suitable cysteine analogues include S-methyl-L cysteine. As with the other amino acids, the amino acid analogues are incorporated into the compositions in either their free base form or their salt form. In a further embodiment of the invention the amino acids or amino acid analogues are used in a concentration, which is sufficient to prevent or delay aggregation of the protein.

In a further embodiment of the invention methionine (or other sulphuric amino acids or amino acid analogous) may be added to inhibit oxidation of methionine residues to methionine sulfoxide when the polypeptide acting as the therapeutic agent is a polypeptide comprising at least one methionine residue susceptible to such oxidation. By "inhibit" is intended minimal accumulation of methionine oxidized species over time. Inhibiting methionine oxidation results in greater retention of the polypeptide in its proper molecular form. Any stereoisomer of methionine (L, D, or DL isomer) or combinations thereof can be used. The amount to be added should be an amount sufficient to inhibit oxidation of the methionine residues such that the amount of methionine sulfoxide is acceptable to regulatory agencies. Typically, this means that the composition contains no more than about 10% to about 30% methionine sulfoxide. Generally, this can be achieved by adding methionine such that the ratio of methionine added to methionine residues ranges from about 1:1 to about 1000:1, such as 10:1 to about 100:1.

In a further embodiment of the invention the formulation further comprises a stabilizer selected from the group of high molecular weight polymers or low molecular compounds. In a further embodiment of the invention the stabilizer is selected from polyethylene glycol (e.g. PEG 3350), polyvinyl alcohol (PVA), polyvinylpyrrolidone, carboxy/hydroxycellulose or derivates thereof (e.g. HPC, HPC-SL, HPC-L and HPMC), cyclodextrins, sulphur-containing substances as monothioglycerol, thioglycolic acid and 2-methylthioethanol, and different salts (e.g. sodium chloride). Each one of these specific stabilizers constitutes an alternative embodiment of the invention.

The pharmaceutical compositions may also comprise additional stabilizing agents, which further enhance stability of a therapeutically active polypeptide therein. Stabilizing agents of particular interest to the present invention include, but are not limited to, methionine and EDTA, which protect the polypeptide against methionine oxidation, and a nonionic surfactant, which protects the polypeptide against aggregation associated with freeze-thawing or mechanical shearing.

In a further embodiment of the invention the formulation further comprises a surfactant. In a further embodiment of the invention the surfactant is selected from a detergent, ethoxylated castor oil, polyglycolyzed glycerides, acetylated monoglycerides, sorbitan fatty acid esters, polyoxypropylene-polyoxyethylene block polymers (eg. poloxamers such as Pluronic® F68, poloxamer 188 and 407, Triton X-100 ), polyoxyethylene sorbitan fatty acid esters, polyoxyethylene and polyethylene derivatives such as alkylated and alkoxylated derivatives (tweens, e.g. Tween-20, Tween-40, Tween-80 and Brij-35), monoglycerides or ethoxylated derivatives thereof, diglycerides or polyoxyethylene derivatives thereof, alcohols, glycerol, lectins and phospholipids (eg. phosphatidyl serine, phosphatidyl choline, phosphatidyl ethanolamine, phosphatidyl inositol, diphosphatidyl glycerol and sphingomyelin), derivates of phospholipids (eg. dipalmitoyl phosphatidic acid) and lysophospholipids (eg. palmitoyl lysophosphatidyl-L-serine and 1-acyl-sn-glycero-3-phosphate esters of ethanolamine, choline, serine or threonine) and alkyl, alkoxyl (alkyl ester), alkoxy (alkyl ether)- derivatives of lysophosphatidyl and phosphatidylcholines, e.g. lauroyl and myristoyl derivatives of lysophosphatidylcholine, dipalmitoylphosphatidylcholine, and modifications of the polar head group, that is cholines, ethanolamines, phosphatidic acid, serines, threonines, glycerol, inositol, and the positively charged DODAC, DOTMA, DCP, BISHOP, lysophosphatidylserine and lysophosphatidylthreonine, and glycerophospholipids (eg. cephalins), glyceroglycolipids (eg. galactopyransoide), sphingoglycolipids (eg. ceramides, gangliosides), dodecylphosphocholine, hen egg lysolecithin, fusidic acid derivatives- (e.g. sodium tauro-dihydrofusidate etc.), longchain fatty acids and salts thereof C6-C12 (eg. oleic acid and caprylic acid), acylcarnitines and derivatives, N□-acylated derivatives of lysine, arginine or histidine, or side-chain acylated derivatives of lysine or arginine, N □-acylated derivatives of dipeptides comprising any combination of lysine, arginine or histidine and a neutral or acidic amino acid, N□-acylated derivative of a tripeptide comprising any combination of a neutral amino acid and two charged amino acids, DSS (docusate sodium, CAS registry no [577-11-7]), docusate calcium, CAS registry no [128-49-4]), docusate potassium, CAS registry no [7491-09-0]), SDS (sodium dodecyl sulphate or sodium lauryl sulphate), sodium caprylate, cholic acid or derivatives thereof, bile acids and salts thereof and glycine or taurine conjugates, ursodeoxycholic acid, sodium cholate, sodium deoxycholate, sodium taurocholate, sodium glycocholate, N-Hexadecyl-N,N-dimethyl-3-ammonio-1-propanesulfonate, anionic (alkyl-aryl-sulphonates) monovalent surfactants, zwitterionic surfactants (e.g. N-alkyl-N,N-dimethylammonio-1-propanesulfonates, 3-cholamido-1-propyldimethylammonio-1-propanesulfonate, cationic surfactants (quaternary ammonium bases) (e.g. cetyl-trimethylammonium bromide, cetylpyridinium chloride), non-ionic surfactants (eg. Dodecyl □-D-glucopyranoside), poloxamines (eg. Tetronic's), which are tetrafunctional block copolymers derived from sequential addition of propylene oxide and ethylene oxide to ethylenediamine, or the surfactant may be selected from the group of imidazoline derivatives, or mixtures thereof. Each one of these specific surfactants constitutes an alternative embodiment of the invention.

The use of a surfactant in pharmaceutical compositions is well-known to the skilled person. For convenience reference is made to Remington: The Science and Practice of Pharmacy, 19th edition, 1995.

It is possible that other ingredients may be present in the peptide pharmaceutical formulation of the present invention. Such additional ingredients may include wetting agents, emulsifiers, antioxidants, bulking agents, tonicity modifiers, chelating agents, metal ions, oleaginous vehicles, proteins (e.g., human serum albumin, gelatine or proteins) and a zwitterion (e.g., an amino acid such as betaine, taurine, arginine, glycine, lysine and histidine). Such additional ingredients, of course, should not adversely affect the overall stability of the pharmaceutical formulation of the present invention.

Pharmaceutical compositions containing IL-20 or any other compound as mentioned above according to the present invention may be administered to a patient in need of such treat-ment at several sites, for example, at topical sites, for example, skin and mucosal sites, at sites which bypass absorption, for example, administration in an artery, in a vein, in the heart, and at sites which involve absorption, for example, administration in the skin, under the skin, in a muscle or in the abdomen.

Administration of pharmaceutical compositions according to the invention may be through several routes of administration, for example, lingual, sublingual, buccal, in the mouth, oral, in the stomach and intestine, nasal, pulmonary, for example, through the bronchioles and alveoli or a combination thereof, epidermal, dermal, transdermal, vaginal, rectal, ocular, for examples through the conjunctiva, uretal, and parenteral to patients in need of such a treatment.

Compositions of the current invention may be administered in several dosage forms, for example, as solutions, suspensions, emulsions, microemulsions, multiple emulsion, foams, salves, pastes, plasters, ointments, tablets, coated tablets, rinses, capsules, for example, hard gelatine capsules and soft gelatine capsules, suppositories, rectal capsules, drops, gels, sprays, powder, aerosols, inhalants, eye drops, ophthalmic ointments, ophthalmic rinses, vaginal pessaries, vaginal rings, vaginal ointments, injection solution, in situ transforming solutions, for example in situ gelling, in situ setting, in situ precipitating, in situ crystallization, infusion solution, and implants.

Compositions of the invention may further be compounded in, or attached to, for example through covalent, hydrophobic and electrostatic interactions, a drug carrier, drug delivery system and advanced drug delivery system in order to further enhance stability of of IL-20 or any other compound as mentioned above, increase bioavailability, increase solubility, decrease adverse effects, achieve chronotherapy well known to those skilled in the art, and increase patient compliance or any combination thereof. Examples of carriers, drug delivery systems and advanced drug delivery systems include, but are not limited to, polymers, for example cellulose and derivatives, polysaccharides, for example dextran and derivatives, starch and derivatives, poly(vinyl alcohol), acrylate and methacrylate polymers, polylactic and polyglycolic acid and block co-polymers thereof, polyethylene glycols, carrier proteins, for example albumin, gels, for example, thermogelling systems, for example block co-polymeric systems well known to those skilled in the art, micelles, liposomes, microspheres, nanoparticulates, liquid crystals and dispersions thereof, L2 phase and dispersions there of, well known to those skilled in the art of phase behaviour in lipid-water systems, polymeric micelles, multiple emulsions, self-emulsifying, self-microemulsifying, cyclodextrins and derivatives thereof, and dendrimers.

Compositions of the current invention are useful in the formulation of solids, semisolids, powder and solutions for pulmonary administration of IL-20 or any other compound as mentioned above using, for example a metered dose inhaler, dry powder inhaler and a nebulizer, all being devices well known to those skilled in the art.

Compositions of the current invention are specifically useful in the formulation of controlled, sustained, protracting, retarded, and slow release drug delivery systems. More specifically, but not limited to, compositions are useful in formulation of parenteral controlled release and sustained release systems (both systems leading to a many-fold reduction in number of administrations), well known to those skilled in the art. Even more preferably, are controlled release and sustained release systems administered subcutaneous. Without limiting the scope of the invention, examples of useful controlled release system and compositions are hydrogels, oleaginous gels, liquid crystals, polymeric micelles, microspheres, nanoparticles,

Methods to produce controlled release systems useful for compositions of the current invention include, but are not limited to, crystallization, condensation, co-crystallization, precipitation, co-precipitation, emulsification, dispersion, high pressure homogenisation, encapsulation, spray drying, microencapsulating, coacervation, phase separation, solvent evaporation to produce microspheres, extrusion and supercritical fluid processes. General reference is made to Handbook of Pharmaceutical Controlled Release (Wise, D.L., ed. Marcel Dekker, New York, 2000) and Drug and the Pharmaceutical Sciences vol. 99: Protein Formulation and Delivery (MacNally, E.J., ed. Marcel Dekker, New York, 2000).

Parenteral administration may be performed by subcutaneous, intramuscular, intraperitoneal or intravenous injection by means of a syringe, optionally a pen-like syringe. Alternatively, parenteral administration can be performed by means of an infusion pump. A further option is a composition which may be a solution or suspension for the administration of IL-20 or any other compound as mentioned above, in the form of a nasal or pulmonal spray. As a still further option, the pharmaceutical compositions containing IL-20 or any other compound as mentioned above can also be adapted to transdermal administration, e.g. by needle-free injection or from a patch, optionally an iontophoretic patch, or transmucosal, e.g. buccal, administration.

The term "stabilized formulation" refers to a formulation with increased physical stability, increased chemical stability or increased physical and chemical stability.

The term "physical stability" of the protein formulation as used herein refers to the tendency of the protein to form biologically inactive and/or insoluble aggregates of the protein as a result of exposure of the protein to thermo-mechanical stresses and/or interaction with interfaces and surfaces that are destabilizing, such as hydrophobic surfaces and interfaces. Physical stability of the aqueous protein formulations is evaluated by means of visual inspection and/or turbidity measurements after exposing the formulation filled in suitable containers (e.g. cartridges or vials) to mechanical/physical stress (e.g. agitation) at different temperatures for various time periods. Visual inspection of the formulations is performed in a sharp focused light with a dark background. The turbidity of the formulation is characterized by a visual score ranking the degree of turbidity for instance on a scale from 0 to 3 (a formulation showing no turbidity corresponds to a visual score 0, and a formulation showing visual turbidity in daylight corresponds to visual score 3). A formulation is classified physically unstable with respect to protein aggregation, when it shows visual turbidity in daylight. Alternatively, the turbidity of the formulation can be evaluated by simple turbidity measurements well-known to the skilled person. Physical stability of the aqueous protein formulations can also be evaluated by using a spectroscopic agent or probe of the conformational status of the protein. The probe is preferably a small molecule that preferentially binds to a non-native conformer of the protein. One example of a small molecular spectroscopic probe of protein structure is Thioflavin T. Thioflavin T is a fluorescent dye that has been widely used for the detection of amyloid fibrils. In the presence of fibrils, and perhaps other protein configurations as well, Thioflavin T gives rise to a new excitation maximum at about 450 nm and enhanced emission at about 482 nm when bound to a fibril protein form. Unbound Thioflavin T is essentially non-fluorescent at the wavelengths.

Other small molecules can be used as probes of the changes in protein structure from native to non-native states. For instance the "hydrophobic patch" probes that bind preferentially to exposed hydrophobic patches of a protein. The hydrophobic patches are generally buried within the tertiary structure of a protein in its native state, but become exposed as a protein begins to unfold or denature. Examples of these small molecular, spectroscopic probes are aromatic, hydrophobic dyes, such as antrhacene, acridine, phenanthroline or the like. Other spectroscopic probes are metal-amino acid complexes, such as cobalt metal complexes of hydrophobic amino acids, such as phenylalanine, leucine, isoleucine, methionine, and valine, or the like.

The term "chemical stability" of the protein formulation as used herein refers to chemical covalent changes in the protein structure leading to formation of chemical degradation products with potential less biological potency and/or potential increased immunogenic properties compared to the native protein structure. Various chemical degradation products can be formed depending on the type and nature of the native protein and the environment to which the protein is exposed. Elimination of chemical degradation can most probably not be completely avoided and increasing amounts of chemical degradation products is often seen during storage and use of the protein formulation as well-known by the person skilled in the art. Most proteins are prone to deamidation, a process in which the side chain amide group in glutaminyl or asparaginyl residues is hydrolysed to form a free carboxylic acid. Other degradation pathways involves formation of high molecular weight transformation products where two or more protein molecules are covalently bound to each other through transamidation and/or disulfide interactions leading to formation of covalently bound dimer, oligomer and polymer degradation products (Stability of Protein Pharmaceuticals, Ahern. T.J. & Manning M.C., Plenum Press, New York 1992). Oxidation (of for instance methionine residues) can be mentioned as another variant of chemical degradation. The chemical stability of the protein formulation can be evaluated by measuring the amount of the chemical degradation products at various time-points after exposure to different environmental conditions (the formation of degradation products can often be accelerated by for instance increasing temperature). The amount of each individual degradation product is often determined by separation of the degradation products depending on molecule size and/or charge using various chromatography techniques (e.g. SEC-HPLC and/or RP-HPLC).

Hence, as outlined above, a "stabilized formulation" refers to a formulation with increased physical stability, increased chemical stability or increased physical and chemical stability. In general, a formulation must be stable during use and storage (in compliance with recommended use and storage conditions) until the expiration date is reached.

A compound of the invention optionally together with the combination agent for use in treating disease or disorders according to the present invention may be administered alone or in combination with pharmaceutically acceptable carriers or excipients, in either single or multiple doses. The formulation of the combination may be as one dose unit combining the compounds, or they may be formulated as seperate doses. The pharmaceutical compositions comprising a compound of the invention optionally together with the combination agent for use in treating neovascularisation according to the present invention may be formulated with pharmaceutically acceptable carriers or diluents as well as any other known adjuvants and excipients in accordance with conventional techniques such as those disclosed above.

### EXAMPLES

The following model is useful for ischemic angiogenesis: Angiogenic synergism, vascular stability and improvement of hind-limb ischemia by a combination of PDGF-BB and FGF-2. Cao RH; Brakenhielm E; Pawliuk R; Wariaro D; Post MJ; Wahlberg E; Leboulch P; Cao YH, Nature Medicine, Vol. 9 (5) 604-613 (2003).

The mouse matrigel plug technique showed that neovascularisation, as determined by haemoglobin content, after one week was significantly increased when IL-20 at 1.7-170 µg/ml but not 0.17 µg/ml was included in the matrigel. Revealed by the technique of haemoglobin determination, the neovascularisation by IL-20 was dependent on the co-administration of heparin into the gel. However, histological examination showed that IL-20 was capable of inducing neovascularisation in the matrigels without co-administration of heparin.

The mouse cornea model revealed that IL-20 was able to induce neovascularisation in a tissue. Implantation of a pellet consisting of IL-20 in a polymer provoked neovascularisation starting from the corneal rim. Evaluation was performed 6 day after implantation by measuring the area covered by new blood vessel.

In-vitro we showed that IL-20 exhibited dose-dependent chemotactic effects on HUVEC in the modified Boyden chamber assay

### MATRIGEL PLUG ASSAY

Groups of 10 animals were injected s.c. 0.25 ml of matrigel at two disticnt sites near the ventral midline and posterior to the xiphoid process. To the matrigel was added different concentrations of IL-20 with or without heparin (15 IU). Matrigel lacking il-20 and/or heparin were injected at control sites. After 7 days animals were euthanized with CO2. The matrigel plugs were removed, weighed and homogenised in two ml of water. The amount of hemoglobulin was subsequently determined by spectrophotometry. Alternatively the plugs were placed intact into paraformaldehyd and after fixation cut into histological slides and stained with hematoxilin / eosine for visual inspection of the vessel growth.

### RESULTS:

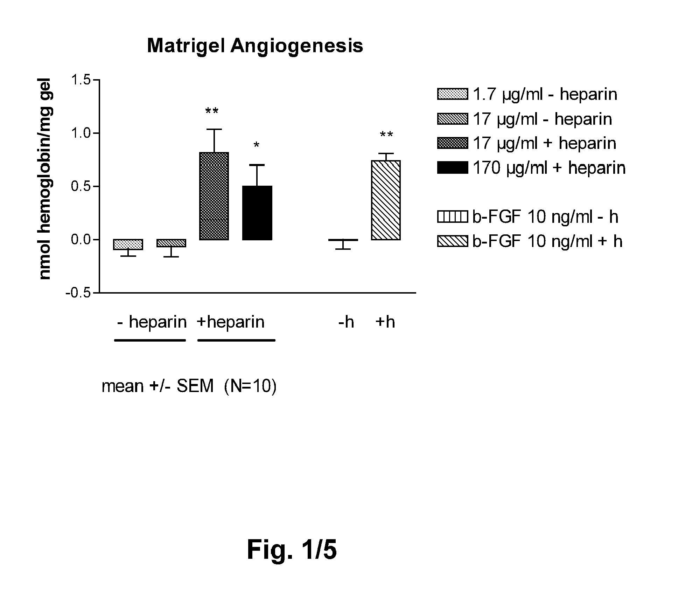

hIL-20 matrigel plugs were implanted into mice in two different concentrations together with empty control gels. Result show that hIL-20 induces an angiogenic response in the mice measured by hemoglobin influx in the gel. Experiment were done with or without heparin and with inclusion of b-FGF as positive control. The angiogenetic effect seems to be dependent on Heparin. Results are shown in Fig.1.

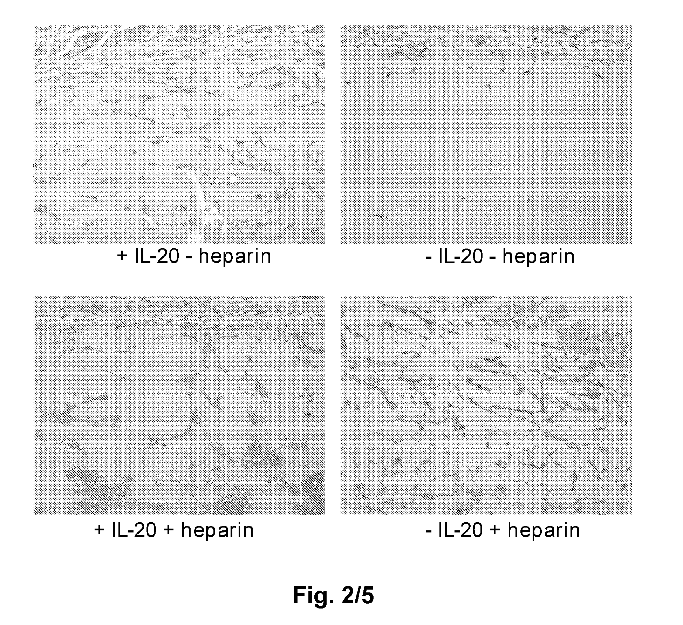

The histological examinations proved intense vessel formations in the matrigel, and this did not require the co-administration with heparin. Results shown in Fig.2.

### MOUSE CORNEAL NEOVASCULARISATION ASSAY:

The mouse corneal assay was performed according to procedures described previously (Cao et al., 1999). Corneal micropockets were created with a modified von Graefe cataract knife in both eyes of male, 5-6-week-old C57BI6/J mice. A micropellet (0.35 X 0.35 mm) of sucrose and aluminium sulfate (Bukh Meditec, Copenhagen, Denmark) coated with hydron polymer type NCC (IFN Sciences, New Brunswick, NJ, USA) containing 600 ng of hIL-20 was implanted into each pocket. The pellet was positioned 0.6-0.8 mm from the corneal limbus. After implantation, erythromycin ophthalmic ointment was applied to each eye. The eyes were examined by a slit-lamp biomicroscope on day 6 after pellet implantation. Vessel length and clock hours of circumferential neovascularization were measured.

### RESULTS:

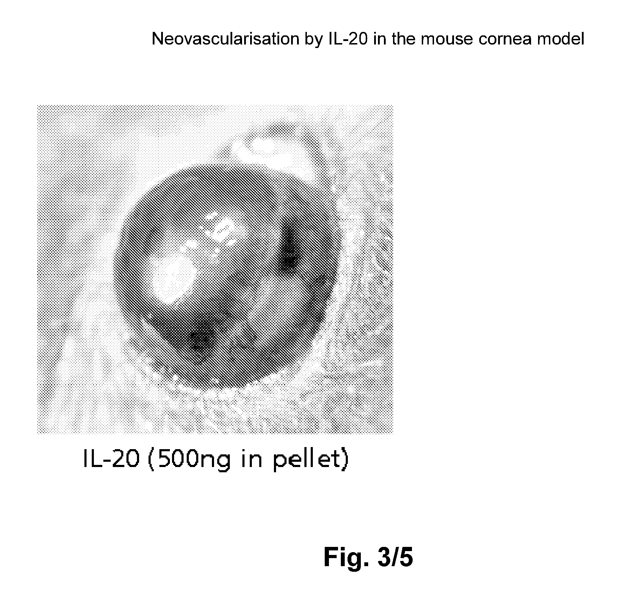

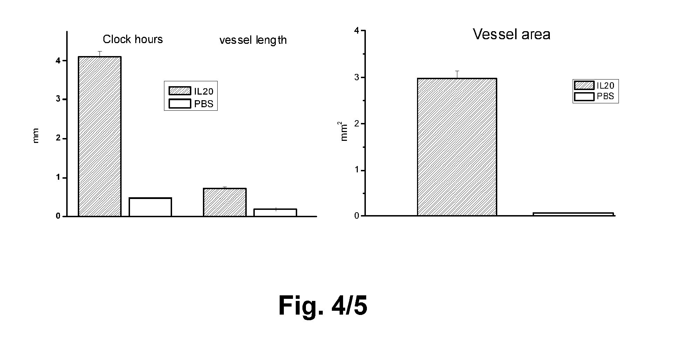

Fig. 3 upper part demonstrates that the presence of a pellet containing IL-20 induced neovascularisation in the cornea. Quantification of the response is shown in figure 4. The pellet containing IL-20 induces a much larger reponse than pellets containing phosphate buffered saline (PBS).

### MIGRATION ASSAY

Chemotactic behaviour of human umbilicord veneous endothelial cells was scrutinized in a modified Boyden chamber (Neuroprobe AP48) using a polycarbonate filter with 12□m pores. Cells were serum starved for 24h (HAM F-12, 2% FBS), before they were placed in the upper chamber (800.000 /ml) while the lower chamber contained HAM F-12 alone (control) or different concentrations of IL-20. Each result was based on the combined values from 2 experiments using 6 wells for each group. After 4h incubation at 37oC and in air containing 5% CO2 the number of cells that migrated to the lower surface of the polycarbonate filter was determined. The filter was taken our of the chamber, fixated in methanol and subsequently the cells were stained with Giemsa. The cells on the upper surface of the filter was removed by scraping with a cotton wick. The cell number was quantified by counting using a microscope.

### RESULTS:

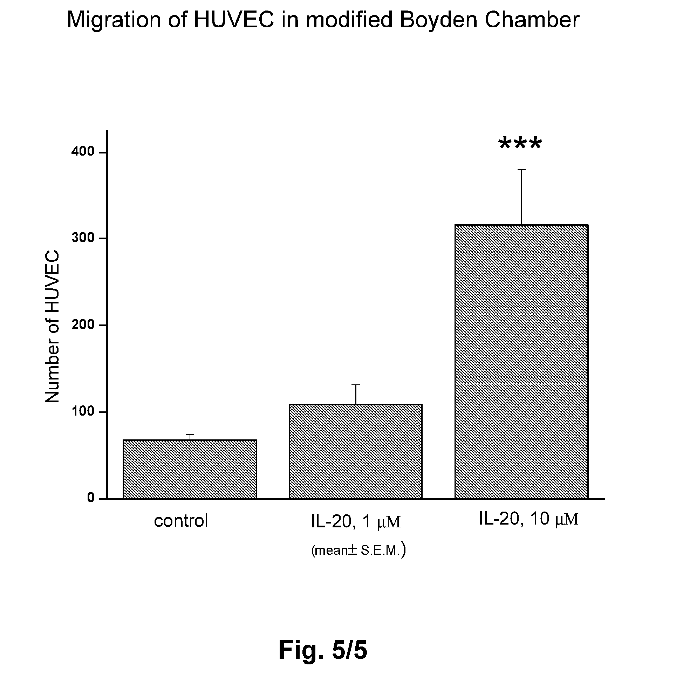

Fig. 5 shows that IL-20 exhibited dose-dependent chemotactic effects on HUVEC. While 1µM of IL-20 only had a modest effect, 10µM of IL-20 clearly increased the number of cells demonstrating that IL-20 is chemotactic for endothelial cells (ANOVA, P< 0.001). The data supporting the in vivo findings, that IL-20 play a role in neovascularisation.

## Claims

1. A method of diagnosing neovascularization activity in rheumatoid arthritis by analyzing the activity of an IL20 polypeptide defined by SEQ ID NO:1 in a tissue sample.

2. A method of diagnosing neovascularization activity in ischemia by analyzing the activity of an IL20 polypeptide defined by SEQ ID NO:1 in a tissue sample.

3. A method of diagnosing neovascularization activity in Crohn's disease by analyzing the activity of an IL20 polypeptide defined by SEQ ID NO:1 in a tissue sample.

4. A method of diagnosing neovascularisation activity in diabetic retinopathy by analyzing the activity of an IL20 polypeptide defined by SEQ ID NO:1 in a tissue sample.

5. A method of diagnosing neovascularization activity in atherosclerosis by analyzing the activity of an IL20 polypeptide defined by SEQ ID NO:1 in a tissue sample.

6. The method of any preceding claim, wherein the activity is detected by use of a labeled antibody.

7. The use of a labeled antibody detecting the activity of an IL20 polypeptide defined by SEQ ID NO:1 in the preparation of a medicament for diagnosing neovascularization activity in rheumatoid arthritis.

8. The use of a labeled antibody detecting the activity of an IL20 polypeptide defined by SEQ ID NO:1 in the preparation of a medicament for diagnosing neovascularization activity in ischemia.

9. The use of a labeled antibody detecting the activity of an IL20 polypeptide defined by SEQ ID NO:1 in the preparation of a medicament for diagnosing neovascularization activity in Crohn's disease.

10. The use of a labeled antibody detecting the activity of an IL20 polypeptide defined by SEQ ID NO:1 in the preparation of a medicament for diagnosing neovascularization activity in diabetic retinopathy.
